# EUROPÄISCHE PATENTANMELDUNG

(11) **EP 1 398 615 A1**
(43) Veröffentlichungstag der Anmeldung: **17.03.2004**
(21) Anmeldenummer: 02019697.8
(22) Anmeldetag: 04.09.2002
(51) Int. Cl.: G01N 11/10, G01N 33/49

(54) **Verfahren und Vorrichtung zur Feststellung der Änderung des Fliessverhaltens einer Flüssigkeit**

(71) Anmelder: Trinity Biotech GmbH, 32657 Lemgo (DE)
(72) Erfinder: Wiehe, Josef, 37671 Höxter (DE)
(74) Vertreter: Dantz, Jan Henning

(57) **Zusammenfassung**

Ein Verfharen und eine Vorrichtung zur Feststellung der Änderung des Fließverhaltens einer Flüssigkeit, insbesondere zur Festellung des Gerinnungsverhaltens von Blut oder Blutplasma, wobei eine ferromagnetische, vollständig in die in eine Küvette gefüllte Flüssigkeit eingetauchte Kugel (2) von einem Magneten (4) gehalten und relativ zur Küvette (1) auf einer bodenseitigen, konzentrischen Umlaufbahn bewegt und danach eine bei Abnahme der Fließfähigkeit veränderte Umlaufbewegung der Kugel (2) erfaßt und ausgewerte wird, ist so ausgebildet, daßdie Kugel (2) reversierend bewegt wird.

## Beschreibung

Die vorliegende Erfindung betrifft ein Verfahren und eine Vorrichtung zur Feststellung der Änderung des Fließverhaltens einer Flüssigkeit gemäß dem Oberbegriff des Anspruchs 1.

Ein derartiges Verfahren sowie eine solche Vorrichtung sind aus der US 5 072 610 bekannt.

Darin wird gezeigt und beschrieben, daß ein konzentrisch sich drehender Finger des unterhalb der Küvette angeordneten rotierenden Magneten die am Boden der feststehenden Küvette liegende Kugel auf ihrer Umlaufbahn mitnimmt, bis durch die einsetzende Gerinnung der in der Küvette befindlichen Blutprobe und der damit sich ausbildende Fibrinfaserbildung die Bewegung der Kugel im Sinne eines Abbremsens verändert wird.

Diese Veränderung wird durch Erfassungseinrichtungen in Form von Sensoren festgestellt, die die Drehung der Kugel und des Magneten miteinander vergleichen. Dabei sind die Sensoren seitlich der Küvette bzw. des Magneten angeordnet.

Allerdings ist die bekannte Vorrichtung aus mehreren Gründen nicht geeignet, eine optimale Untersuchung des Blutes zu realisieren.

Zunächst einmal ist bauartbedingt ein erheblicher Platz zur Unterbringung der Erfassungseinrichtungen erforderlich, wodurch beispielsweise verhindert wird, daß Küvettenplatten zum Einsatz kommen, bei denen eine Mehrzahl von Küvetten in Reihe angeordnet in einer Platte integriert sind. Da dabei die Abstände der einzelnen Küvetten zueinander sinnvollerweise relativ gering gehalten werden sollen, um so eine maximale Anzahl von Küvetten unterbringen zu können, ist deren Einsatz für die bekannte Vorrichtung prinzipiell wenig geeignet. Insbesondere deshalb, weil jeder der in einer Reihe nebeneinander liegenden Küvetten ein Magnet und die dazu gehörenden Erfassungseinrichtungen zugeordnet sein müssen.

Eine rationelle Untersuchung, also die gleichzeitige Untersuchung mehrerer Proben ist praktisch nicht möglich.

Darüber hinaus läßt auch die Genauigkeit der Untersuchung zu wünschen übrig, da eine Erkennung der Änderung des Fließverhaltens der Flüssigkeit, sprich des Blutes, unter Umständen erst nach einer nahezu vollständigen Umdrehung des Magneten möglich ist. Erst dann ist definitiv erkennbar, daß die Kugel gegenüber dem Magneten nachläuft.

Hierdurch kann es, wenn auch nur zu geringen, aber immerhin doch zu Verfälschungen des Untersuchungsergebnisses kommen, d. h. also im konkreten Fall, um eine Fehlinterpretation des Gerinnungsverhaltens des Blutes.

Der vorliegenden Erfindung liegt daher die Aufgabe zugrunde, ein Verfahren sowie eine Vorrichtung der jeweils gattungsgemäßen Art so auszubilden, daß eine rationellere und genauere Feststellung der Änderung des Fließverhaltens einer Flüssigkeit möglich ist.

Diese Aufgabe wird durch ein Verfahren gelöst mit den Merkmalen des Anspruchs 1 und durch eine Vorrichtung mit den Merkmalen des Anspruches 6.

Im Gegensatz zum beschriebenen Stand der Technik wird durch das neue Verfahren bzw. die neue Vorrichtung eine Veränderung des Magnetfeldes erkannt, die durch die Lageveränderung der Kugel gegenüber dem Magneten eintritt.

Dabei ist die momentane Position der Kugel unerheblich. Die Lageveränderung, also eine Abbremsung durch die sich bildenden Fibrinfasern bei einer Blutgerinnung, wird sofort und unmittelbar erkannt, so daß sich eine wesentliche Verbesserung des Verfahrens zur Feststellung des Gerinnungsverhaltens von Blut oder Blutplasma ergibt.

Als weiterer Vorteil der Vorrichtung ist zu bemerken, daß die Erfassungseinrichtung nun unterhalb der Küvette plaziert ist, wodurch sich hinsichtlich des Platzbedarfes eine wesentliche Verbesserung gegenüber dem Stand der Technik ergibt.

Da die Erfassungseinrichtung nun nicht mehr seitlich angeordnet ist, kann der freie Platz genutzt werden, um hier eine weitere Spule mit zugeordnetem Magneten und eine Erfassungseinrichtung oder anderen Untersuchungskomponenten zu installieren.

Hierdurch können die erwähnten Küvettenplatten eingesetzt werden, die eine sehr rationelle Untersuchung des Blutes erlauben, da gleichzeitig eine Mehrzahl von Untersuchungen möglich ist.

Insbesondere unter dem Aspekt, daß die in Rede stehenden Blutuntersuchungen in großem Umfang durchgeführt werden, kommt den genannten Vorteilen eine besondere Bedeutung zu.

Erfindungsgemäß ist die Erfassungseinrichtung zwischen der Küvette und der Antriebsspule angeordnet und bildet dabei in baulicher Hinsicht praktisch eine Verlängerung der Antriebsspule, wobei der Magnet von der Erfassungseinrichtung, die durch eine ringförmige Sensorspule gebildet wird, umschlossen wird.

Alternativ bildet die Antriebsspule selbst die Erfassungseinrichtung. Dabei wird mittels eines angeschlossenen Rechners, ebenso wie dies beim Einsatz einer Sensorspule der Fall ist, die Veränderung des Magnetfeldes bei der Lageveränderung der Kugel erkannt und an eine Auswertelektronik weitergegeben.

Im übrigen ist die Erfindung geeignet, eine solche Vorrichtung besonders preiswert herzustellen, da im Fall des Einsatzes einer Sensorspule diese sehr einfach herzustellen ist, während dann, wenn die Erfassungseinrichtung durch die Spule selbst gebildet wird, im wesentlichen gar keine baulichen Veränderungen bzw. Zusätze erforderlich sind, sondern lediglich ein an sich bereits vorhandener Rechner bzw. die Software dazu entsprechend zu modifizieren ist.

Gemäß dem erfindungsgemäßen Verfahren wird die Kugel reversierend bewegt. Grundsätzlich besteht die Möglichkeit, die Küvette entsprechend zu drehen. Es hat sich jedoch gezeigt, daß die Rotation des Magneten und damit der Kugel in der Praxis sinnvoller ist.

Die reversierende Bewegung erfolgt durch eine erste Drehung um ca. 90° und eine anschließende Zurückdrehung um 60°, so daß sich insgesamt eine schrittweise Rotation ergibt.

Aufgrund ihrer Massenträgheit kann die Kugel bei der Rückwärtsdrehung des Magneten nicht unmittelbar erfolgen, so daß sich der Magnet kurzzeitig von der Kugel entfernt. Hierdurch wird eine Induktionsspannung erzeugt.

Die Drehgeschwindigkeit des Magneten und seine Haltekraft, d. h. sein Magnetfeld, sind so abgestimmt, daß die Kugel eine Eigenschwingung ausführt. Durch das sozusagen Überschwingen der Kugel beim Halten des Magneten wird, wie erwähnt, eine Induktion erzeugt, die in ihrer Amplitude gleich der des Entfernens des Magneten von der Kugel ist.

Dieser Spannungsverlauf wird in einem Analog-Digital-Wandler in digitale Werte umgewandelt. Durch Vergleich der Kurvenverläufe kann exakt der Endzeitpunkt der Messung bestimmt werden, d. h., die Veränderung der Induktionsspannung aufgrund der Veränderung des Fließverhaltens der Flüssigkeit und damit einer Abbremsung der Kugel ist genau erkennbar.

Eine Vorrichtung zur Durchführung des Verfahrens nach Anspruch 1 ist so ausgebildet, daß der Magnet oder die Küvette mittels eines Schrittmotors antreibbar sind.

Im übrigen beschränkt sich der Einsatz des Verfahrens und der Vorrichtung nicht auf die Feststellung einer Änderung des Gerinnungsverhaltens von Blut oder Blutplasma, sondern kann durchaus auch Verwendung finden bei der Verarbeitung von Milch, hier insbesondere bei deren Gerinnung zur Käseproduktion oder der Feststellung einer Viskositätsänderung von Öl bei Temperaturveränderungen.

Weitere vorteilhafte Ausbildungen der Erfindung sind in den Unteransprüchen gekennzeichnet.

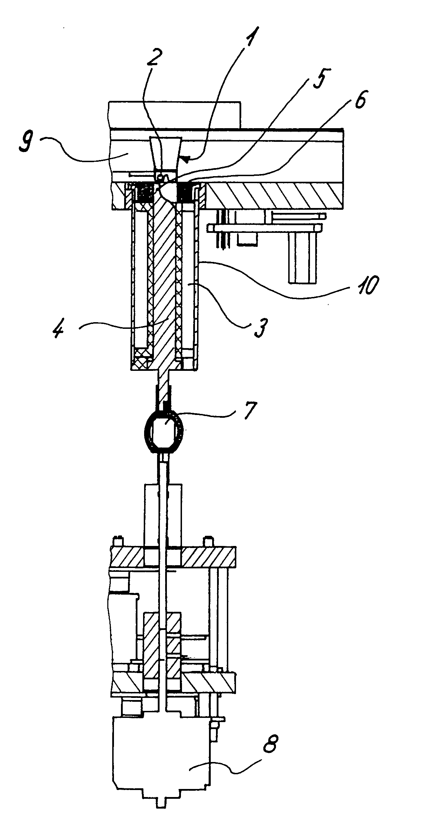

Ein Ausführungsbeispiel der Erfindung wird nachfolgend anhand der beigefügten Zeichnung beschrieben.

Die einzige Figur zeigt eine erfindungsgemäße Vorrichtung in einem Längsschnitt.

In der Figur ist eine Vorrichtung zur Feststellung des Gerinnungsverhaltens von Blut oder Blutplasma dargestellt, die eine Aufnahme 9 aufweist, in der eine Küvette 1 fest positionierbar ist.

Diese dient der Aufnahme von Blut oder Blutplasma sowie entsprechender Reagenzien. Sie weist auf einer bodenseitigen, konzentrischen Umfangsbahn eine drehbare ferromagnetische, in die Flüssigkeit vollständig eingetauchte Kugel 2 auf. Unterhalb des Küvettenbodens, in Verlängerung der Längsachse der Küvette 1, ist zentrisch dazu ein von einer Antriebsspule 3 erregter Magnet 4 angeordnet, der über eine elastische Kupplung 7 mit einem Antriebsmotor 8 verbunden ist und durch diesen in Rotation versetzbar ist.

Auf seiner der Küvette 1 zugewandten Seite weist der Magnet 4 einen eine konzentrische Drehbewegung ausführenden Finger 5 auf, wobei die Drehbahn dieses Fingers 5 der Umlaufbahn der Kugel 2 entspricht. Diese steht mit dem Finger 5 so in Wirkverbindung, daß sie bei dessen Drehung durch die Magnetkräfte mitgenommen wird, während die Küvette 1 fest steht.

Im Bereich des Fingers 5 wird der Magnet 4 von einer ringförmigen Sensorspule 6 umschlossen, die eine Erfassungseinrichtung darstellt.

Dabei kann die Sensorspule 6 an einen von der Antriebsspule 3 unabhängigen Stromkreis angeschlossen sein.

Sobald sich eine Änderung des Fließverhaltens der Flüssigkeit in der Küvette 1, d. h. in diesem Fall das Fließverhalten des Blutes durch Gerinnung ergibt, wird die Mitnahme der Kugel durch den Finger 5 des Magneten 4 abgebremst und das durch die Sensorspule 6 aufgebaute Magnetfeld verändert.

Diesen Zustand erfaßt ein nicht dargestellter Rechner, der an eine ebenfalls nicht gezeigte Auswertelektronik angeschlossen ist, mit deren Hilfe das Gerinnungsverhalten des Blutes definiert wird.

Die ringförmige Sensorspule 6, die Antriebsspule 3 und der Magnet 4 sind in einer Hülse 10 angeordnet, die als eine Baueinheit in die Aufnahme 9 eingesetzt ist, wobei die Sensorspule 6 auf ihrer der Küvette 1 zugewandten Seite etwa flächenbündig mit dem Finger 5 des Magneten 4 abschließt.

Grundsätzlich besteht auch die Möglichkeit der kinematischen Umkehr, wobei dann die Küvette 1 rotiert, während die Kugel 2 von dem dann fest stehenden Magneten 4 in einer fixen Position gehalten wird, die sich erst durch Mitnahme der Kugel bei Gerinnung des Blutes verändert.

### Bezugszeichenliste

- 1: Küvette
- 2: Kugel
- 3: Antriebsspule
- 4: Magnet
- 5: Finger
- 6: Sensorspule
- 7: Kupplung
- 8: Antrieb
- 9: Aufnahme
- 10: Hülse

## Patentansprüche

1. Verfahren zur Feststellung der Änderung des Fließverhaltens einer Flüssigkeit, insbesondere zur Feststellung des Gerinnungsverhaltens von Blut oder Blutplasma, wobei eine ferromagnetische, vollständig in die in eine Küvette gefüllte Flüssigkeit eingetauchte Kugel (2) von einem Magneten (4) gehalten und relativ zur Küvette (1) auf einer bodenseitigen, konzentrischen Umlaufbahn bewegt und danach eine bei Abnahme der Fließfähigkeit veränderte Umlaufbewegung der Kugel (2) erfaßt und ausgewertet wird, **dadurch gekennzeichnet, daß** die Kugel (2) reversierend bewegt wird.

2. Verfahren nach Anspruch 1, **dadurch gekennzeichnet, daß** die Kugel (2) in einem größeren Winkelbereich hin als zurück bewegt wird.

3. Verfahren nach Anspruch 2, **dadurch gekennzeichnet, daß** die Kugel (2) um 90° hin und um 60° zurück bewegt wird.

4. Verfahren nach Anspruch 1, **dadurch gekennzeichnet, daß** eine beim Zurückdrehen des Magneten (4) und der Kugel (2) sich ergebende Induktionsspannung ermittelt und mit einem vorgegebenen Soll-Wert oder einer bei einer vorangegangenen Messung ermittelten verglichen wird.

5. Vorrichtung zur Durchführung des Verfahrens nach Anspruch 1, bei der in einer Flüssigkeit aufnehmenden Küvette (1) auf einer bodenseitigen, konzentrischen Umfangsbahn relativ zur Küvette (1) drehbare ferromagnetische, in die Flüssigkeit vollständig eingetauchte Kugel (2) angeordnet ist, die mittels eines durch eine Antriebsspule (3) erregten Magneten (4) gehalten ist und mit einer Erfassungseinrichtung (6), mit der eine durch eine Abnahme der Fließfähigkeit der Flüssigkeit bewirkte Veränderung der relativen Drehung der Kugel (2) zur Küvette (1) feststellbar ist, **dadurch gekennzeichnet, daß** der Magnet (4) oder die Küvette (1) mittels eines reversierend drehbaren Schrittmotors antreibbar sind.

6. Vorrichtung zur Feststellung der Änderung des Fließverhaltens einer Flüssigkeit, insbesondere zur Feststellung des Gerinnungsverhaltens von Blut oder Blutplasma mit einer die Flüssigkeit aufnehmenden Küvette (1), in der eine auf einer bodenseitigen, konzentrischen Umfangsbahn relativ zur Küvette (1) drehbare ferromagnetische, in die Flüssigkeit vollständig eingetauchte Kugel (2) angeordnet ist, die mittels eines durch eine Antriebsspule (3) erregten Magneten (4) gehalten ist und mit einer Erfassungseinrichtung, mit der eine durch eine Abnahme der Fließfähigkeit der Flüssigkeit bewirkte Veränderung der relativen Drehung der Kugel (2) zur Küvette (1) feststellbar ist, **dadurch gekennzeichnet, daß** die Erfassungseinrichtung zwischen der Küvette (1) und der Antriebsspule (3) angeordnet ist oder durch die Antriebsspule (3) selbst gebildet wird.

7. Vorrichtung nach Anspruch 6, **dadurch gekennzeichnet, daß** die Erfassungseinrichtung als ringförmige Sensorspule (6) ausgebildet ist, die den Magneten (4) in seinem der Küvette (1) zugewandten Endbereich umschließt.

8. Vorrichtung nach Anspruch 7, **dadurch gekennzeichnet, daß** die Sensorspule (6) und die Antriebsspule (3) in einer Hülse (10) plaziert sind, die gemeinsam mit dem Magneten (4) eine Baueinheit bilden.

9. Vorrichtung nach Anspruch 7, **dadurch gekennzeichnet, daß** die Sensorspule (6) flächenbündig mit dem Magneten (4) abschließt.

10. Vorrichtung nach Anspruch 6, **dadurch gekennzeichnet, daß** die Erfassungseinrichtung (6) und/oder die Antriebsspule (3) an einen Rechner angeschlossen ist, mit dem eine mit der Lageveränderung der Kugel (2) einhergehende Veränderung des Magnetfeldes der Erfassungseinrichtung (6) feststellbar ist.
